(19) Europäisches Patentamt
European Patent Office
Office européen des brevets

(11) Veröffentlichungsnummer: **0 316 845 B1**

(12) **EUROPÄISCHE PATENTSCHRIFT**

(45) Veröffentlichungstag der Patentschrift: **19.01.94**

(21) Anmeldenummer: **88118957.5**

(22) Anmeldetag: **14.11.88**

(51) Int. Cl.5: **C07D 487/04**, A01N 43/90, //(C07D487/04,239:00,239:00), (C07D487/04,239:00,235:00)

# (54) 1,2,3,4-Tetrahydro-5-nitro-pyrimidin-Derivate.

(30) Priorität: **18.11.87 DE 3739263**

(43) Veröffentlichungstag der Anmeldung:
**24.05.89 Patentblatt 89/21**

(45) Bekanntmachung des Hinweises auf die Patenterteilung:
**19.01.94 Patentblatt 94/03**

(84) Benannte Vertragsstaaten:
**BE CH DE FR GB IT LI NL**

(56) Entgegenhaltungen:
**EP-A- 0 247 477**
**US-A- 4 031 087**

(73) Patentinhaber: **BAYER AG**

**D-51368 Leverkusen(DE)**

(72) Erfinder: **Gesing, Ernst, Dr.**
**Trillser Graben 4**
**D-4006 Erkrath-Hochdahl(DE)**
Erfinder: **Wolf, Hilmar, Dr.**
**Haus Gravener Strasse 105**
**D-4018 Langenfeld(DE)**
Erfinder: **Becker, Benedikt, Dr.**
**Metzkausener Strasse 14**
**D-4020 Mettmann(DE)**
Erfinder: **Homeyer, Bernhard, Dr.**
**Obere Strasse 28**
**D-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue 1,2,3,4-Tetrahydro-5-nitro-pyrimidin-Derivate, ein Verfahren zu ihrer Herstellung und ihre Verwendung in Schädlingsbekämpfungsmitteln, insbesondere als Insektizide.

Es ist bereits bekannt, daß bestimmte Pyrimidino-thiazine wie z. B. 7-Ethyl-9-nitro-3,4,7,8-tetrahydro-(2H,6H)-pyrimidino-[4,3-b]-1,3-thiazin insektizide Eigenschaften aufweisen (vergl. US-PS 4 031 087).

Die vorliegende Erfindung betrifft:

1,2,3,4-Tetrahydro-5-nitropyrimidin-Derivate der Formel (I)

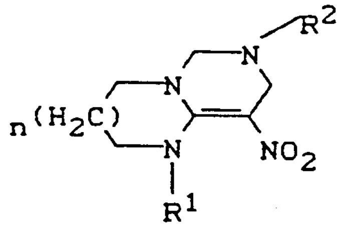

(I)

in welcher

$R^1$ für Wasserstoff oder für gegebenenfalls ein- bis dreifach gleich oder verschieden durch Halogen und/oder Cyano substituierte Reste aus der Reihe Benzyl, Picolyl (Pyridylmethyl) und Phenoxyalkyl steht,

n für die Zahl 0 oder 1 steht und

$R^2$ für die Gruppe -A-$R^3$ steht,
in welcher
A für eine direkte Bindung oder für die Gruppierungen $-(CH_2)_m-$ oder $-(CH_2)_x-Y-(CH_2)_z-$ steht,
worin

m für die Zahlen 1 bis 4 steht,

x und z für die Zahlen 0, 1 oder 2 stehen, wobei

x und z gleich oder verschieden sein können und

Y für Sauerstoff, Schwefel oder für die Gruppierungen -NH- oder

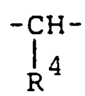

steht
worin

$R^4$ für gegebenenfalls durch Alkoxycarbonyl substituiertes $C_1$-$C_4$-Alkyl, Cyano, Hydroxy oder für Phenyl steht und

$R^3$ für gegebenenfalls einfach bis fünffach, gleich oder verschieden substituiertes Phenyl steht, wobei als Substituenten genannt seien Halogen, $C_{1-4}$-Alkyl, Halogen-$C_{1-2}$-alkyl, $C_{1-2}$-alkoxy, Halogen-$C_{1-2}$-alkylthio, Hydroxy, Di-$C_{1-2}$-alkylamino, Carboxyl und Phenyl,

ausgenommen die Verbindungen, in denen

α)

$R^1$ für den Rest 2-Chlor-pyridin-5-yl-methyl und

$R^2$ gleichzeitig für die folgenden Reste steht: Benzyl, 4-Chlorbenzyl, 2-Chlorbenzyl, Phenethyl, 1-Phenylethyl und 3,4-Dimethoxybenzyl, oder

β)

$R^1$ für Wasserstoff steht und gleichzeitig $R^2$ Benzyl und 4-Chlorbenzyl bedeutet,

und deren Säureadditionssalze.

Bevorzugt sind 1,2,3,4-Tetrahydro-5-nitropyrimidin-Derivate der Formel (I)

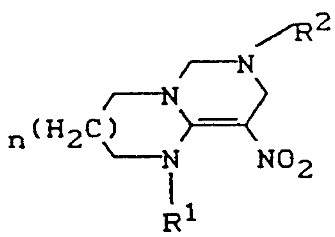

(I)

in welcher

$R^1$ für Wasserstoff oder für gegebenenfalls ein bis dreifach gleich oder verschieden durch Halogen und/oder Cyano substituierte Reste aus der Reihe Benzyl, Picolyl (Pyridylmethyl) und Phenoxyalkyl steht,

n für die Zahl 0 oder 1 steht und

$R^2$ für die Gruppe $-A-R^3$ steht,
in welcher
A für eine direkte Bindung oder für die Gruppierung $-(CH_2)_x-Y-(CH_2)_z-$steht,
worin

x und z für die Zahlen 0, 1 oder 2 stehen, wobei

x und z gleich oder verschieden sein können und

Y für Sauerstoff, Schwefel oder für die Gruppierungen -NH- oder

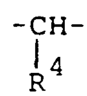

steht worin

$R^4$ für $C_1$-$C_4$-Alkyl oder für Phenyl steht und

$R^3$ für gegebenenfalls durch Halogen oder Phenyl substituiertes Phenyl steht,
ausgenommen die Verbindungen, in denen

$R^1$ für den Rest 2-Chlor-pyridin-5-yl-methyl und

$R^2$ gleichzeitig für 1-Phenylethyl steht,

und deren Säureadditionssalze.

Weiter sind bevorzugt 1,2,3,4-Tetrahydro-5-nitropyrimidin-Derivate der Formel (I) gemäß 1 (oben):
in welcher

n für die Zahl 0 steht,

$R^1$ für Wasserstoff oder für gegebenenfalls einfach bis dreifach, gleich oder verschieden durch Halogen und oder Cyano substituierte Reste aus der Reihe Benzyl, Picolyl und Phenoxy-$C_1$-$C_3$-alkyl steht,

$R^2$ für die Gruppe $-A-R^3$ steht,
in welcher

A für die Gruppierung $-(CH_2)_x-Y-(CH_2)_z-$ steht,
worin

x und z für die Zahlen 0, 1 oder 2 stehen, wobei x und z gleich oder verschieden sein können und

Y für Sauerstoff, Schwefel oder für die Gruppierungen -NH- oder

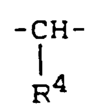

steht
worin

$R^4$ für $C_1$-$C_4$-Alkyl oder für Phenyl steht und

$R^3$ für gegebenenfalls einfach bis fünffach durch Halogen oder Phenyl gleich oder verschieden substituiertes Phenyl steht,

ausgenommen Verbindungen, in denen $R^1$ für 2-Chlor-pyridin-5-yl-methyl und $R^2$ gleichzeitig für 1-Phenylethyl steht sowie

1,2,3,4-Tetrahydro-5-nitro-pyrimidin-Derivate der Formel (I) gemäß 1 (oben),

in welcher

n für die Zahl 1 steht,

$R^1$ für Wasserstoff, gegebenenfalls einfach bis dreifach, gleich oder verschieden durch Halogen und/oder Cyano substituierte Reste aus der Reihe Benzyl, Picolyl und Phenoxy-$C_1$-$C_3$-alkyl steht,

$R^2$ für die Gruppe -A-$R^3$ steht,
in welcher

A für die Gruppierung $-(CH_2)_x-Y(CH_2)_z-$ steht,
worin

x und z für die Zahlen 0, 1 oder 2 stehen, wobei x und z gleich oder verschieden sein können und

Y für Sauerstoff, Schwefel oder für die Gruppierungen -NH- oder

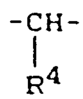

steht
worin

$R^4$ für $C_1$-$C_4$-Alkyl oder für Phenyl steht und

$R^3$ für Halogen-$C_1$-$C_3$-alkyl oder für gegebenenfalls einfach bis fünffach durch Halogen oder Phenyl gleich oder verschieden substituiertes Phenyl steht,

ausgenommen Verbindungen, in denen $R^1$ für 2-Chlor-pyridin-5-yl-methyl und $R^2$ gleichzeitig für 1-Phenylethyl steht.

Besonders bevorzugt sind 1,2,3,4-Tetrahydro-5-nitro-pyrimidin-Derivate der Formel (I) gemäß 1 (oben), in welcher

n für die Zahl 0 oder 1 steht,

$R^1$ für Wasserstoff, gegebenenfalls einfach bis dreifach, gleich oder verschieden durch Halogen und/oder Cyano substituierte Reste aus der Reihe Benzyl, Picolyl und Phenoxy-$C_1$-$C_3$-alkyl steht und

$R^2$ für gegebenenfalls einfach bis fünffach durch Halogen oder Phenyl gleich oder verschieden substituiertes Phenyl steht.

Besonders bevorzugt sind auch

1,2,3,4-Tetrahydro-5-nitro-pyrimidin-Derivate der Formel (I) gemäß (oben),

in welcher

n für die Zahl 0 oder 1 steht,

$R^1$ für Wasserstoff, gegebenenfalls einfach oder zweifach, gleich oder verschieden durch Fluor, Chlor, Brom und/oder Cyano substituierte Reste aus der Reihe Benzyl, Picolyl und Phenoxy-$C_1$-$C_2$-alkylsteht und

$R^2$ für die Gruppe -A-$R^3$ steht,
in welcher

A für die Gruppierung $-(CH_2)_x-Y-)CH_2)_z-$ steht,
worin

x und z für die Zahlen 0, 1 oder 2 stehen, wobei x und z gleich oder verschieden sein können und

Y für Sauerstoff, Schwefel oder für die Gruppierungen -NH- oder

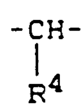

steht

worin

$R^4$ für $C_1$-$C_2$-Alkyl oder für Phenyl steht und

$R^3$ für gegebenenfalls einfach bis fünffach, durch Halogen oder Phenyl gleich oder verschieden substituiertes Phenyl steht.

Einige der neuen 1,2,3,4-Tetrahydro-5-nitro-pyrimidin-Derivate der Formel (I) besitzen ein asymmetrisch substituiertes Kohlenstoffatom und können deshalb in den beiden optischen Isomerenformen anfallen. Die Erfindung betrifft sowohl die Isomerengemische als auch die einzelnen Isomeren.

Weiterhin wurde gefunden, daß man die 1,2,3,4-Tetrahydro-5-nitro-pyrimidin-Derivate der Formel (I) und deren Säureadditions-Salze erhält, wenn man Nitromethylen-Derivate der Formel (II)

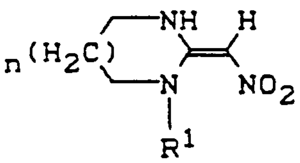

(II)

in welcher

$R^1$ und n die oben angegebenen Bedeutungen haben,

mit Aminen der Formel (III)

$R^3$-A-$NH_2$ (III)

in welcher

$R^3$ und A die oben angegebenen Bedeutungen haben,

in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen physiologisch verträgliche Säuren addiert.

Überraschenderweise zeichnen sich die erfindungsgemäßen 1,2,3,4-Tetrahydro-5-nitro-pyrimidin-Derivate der Formel (I) in überragender Weise durch eine hohe Wirksamkeit als Insektizide aus.

Bevorzugte erfindungsgemäße Verbindungen sind auch Additionsprodukte aus Säuren und denjenigen 1,2,3,4-Tetrahydro-5-nitro-pyrimidin-Derivaten der Formel (I), in denen die Substituenten R, $R^1$, $R^2$ oder der Index n die Bedeutungen haben, die bereits vorzugsweise für diese Substituenten und den Index genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfonsäure, Nikotinsäure, Citronensäure und Ascorbinsäure.

Verwendet man beispielsweise für das erfindungsgemäße Verfahren 3-(2-Chlor-pyridin-5-yl-methyl)-2-nitro-methylen-imidazolidin, 4-Fluorbenzylamin und mindestens die zweifache molare Menge Formaldehyd als Ausgangsstoffe, so kann die entsprechende Reaktion durch das folgende Formelschema wiedergegeben werden:

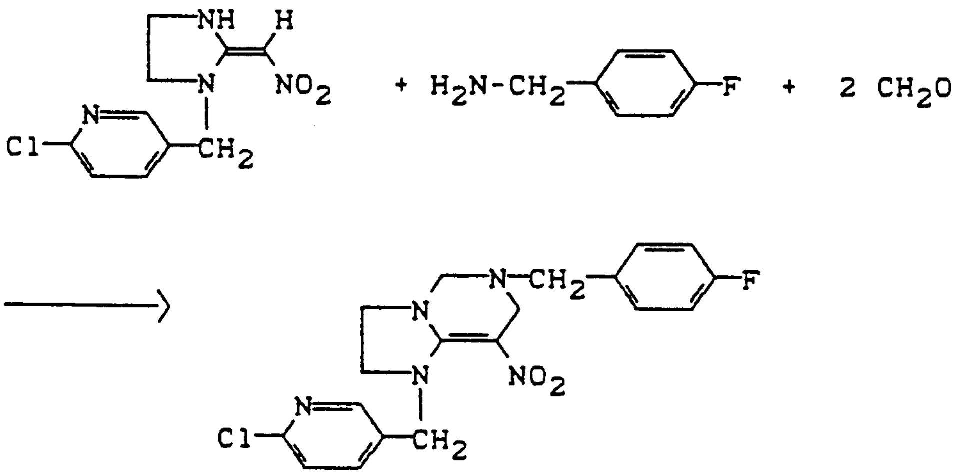

Die beim erfindungsgemäßen Verfahren als Ausgangsstoffe zu verwendenden Nitromethylen-Derivate sind durch die Formel (II) allgemein definiert. In dieser Formel (II) stehen $R^1$ und n vorzugsweise für diejenigen Reste, die bereits im Zusammenhang mit der Beschreibung der erfindungsgemäßen Stoffe der Formel (I) vorzugsweise für diesen Substituenten und den Index n genannt wurden.

Die Verbindungen der Formel (II) sind bekannt und/oder lassen sich nach bekannten Methoden herstellen (vergl. z. B. DE-OS 2 514 402, EP-OS 136 636, EP-OS 154 178 und EP-OS 163 855).

Die beim erfindungsgemäßen Verfahren außerdem als Ausgangsstoffe zu verwendenden Amine sind durch die Formel (III) allgemein definiert. In dieser Formel (III) stehen $R^3$ und A vorzugsweise für diejenigen Reste, die bereits im Zusammenhang mit der Beschreibung der erfindungsgemäßen Stoffe der Formel (I) vorzugsweise für diese Substituenten genannt wurden.

Die Amine der Formel (III) sind allgemein bekannte Verbindungen der organischen Chemie.

Als Beispiele für die Verbindungen der Formel (III) seien genannt:

$R^3\text{-A-}NH_2$ (III)

**Tabelle 1**

| A | $R^3$ |
|---|---|
| $-CH_2-$ | 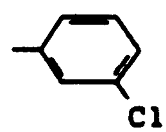 |
| $-CH_2-$ | 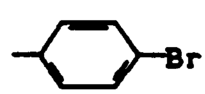 |
| $-CH_2-$ | 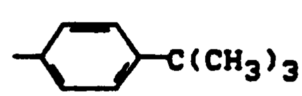 |

**Tabelle 1** - Fortsetzung

| A | $R^3$ |
|---|---|
| $-CH_2-$ | 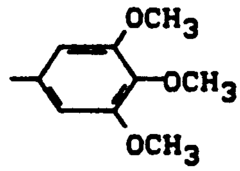 |
| $-CH_2-$ | 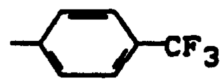 |
| $-CH_2-$ | 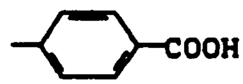 |
| $-CH_2-$ | 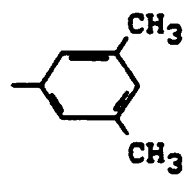 |
| $-CH_2-CH_2-$ | 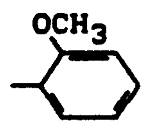 |
| $-CH_2-CH_2-$ | 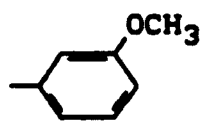 |
| $-CH_2-CH_2-$ | 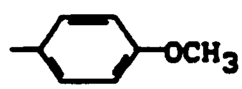 |
| $-CH_2-CH_2-$ | 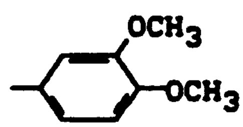 |

<u>Tabelle 1</u> - Fortsetzung

| A | $R^3$ |
|---|---|
| $-CH_2-CH_2-$ | 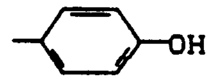 |
| $-CH_2-CH_2-$ | 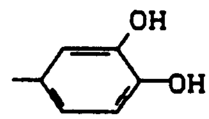 |
| $-CH_2-CH_2-$ | 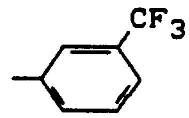 |
| OH<br>\|<br>$-CH-CH_2-$ | 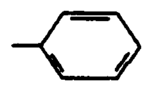 |
| $-(CH_2)_3-$ | 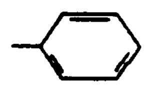 |
| $CH_3$<br>\|<br>$-CH-(CH_2)_2-$ | 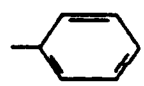 |
| $-(CH_2)_4-$ | 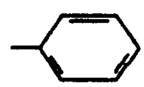 |
| $-(CH_2)_2-CH-$<br>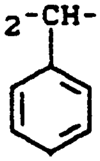 | 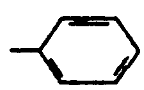 |

Tabelle 1 - Fortsetzung

| A | $R^3$ |
|---|---|
| 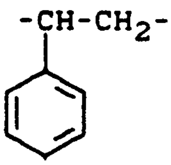 | 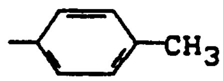 |
| 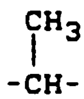 | 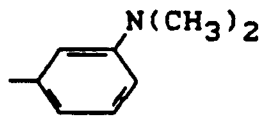 |
| 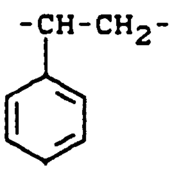 | 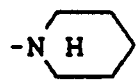 |
| 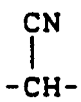 | 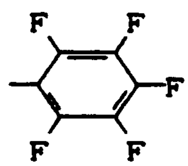 |
| 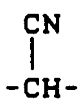 | 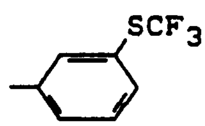 |
| 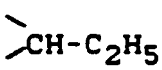 | 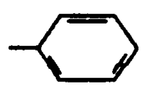 |
| 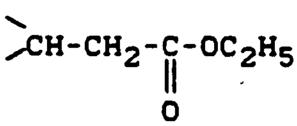 | 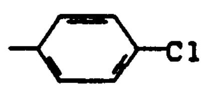 |

Tabelle 1 - Fortsetzung

| A | $R^3$ |
|---|---|
| 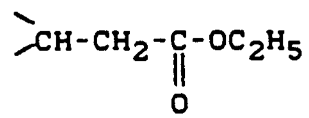 | 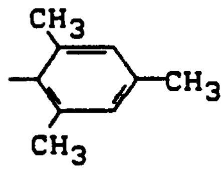 |
| 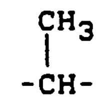 | 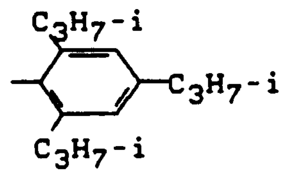 |

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei Wasser und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesetzt.

Das erfindungsgemäße Verfahren wird gegebenenfalls in Gegenwart von sauren, nicht oxidierenden Katalysatoren durchgeführt. Besonders bewährt haben sich Halogenwasserstoffsäuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren in einem größeren Bereich variiert werden. Im allgemeinen arbeitet man bei Temperaturen zwischen -20 °C und +120 °C, vorzugsweise bei Temperaturen zwischen 0 °C und +80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls als wäßrige Lösungen eingesetzt werden. Bei der Verwendung von gasförmigen Aminen der Formel (III) können diese Verbindungen durch das Gemisch aus Verdünnungsmittel, Verbindungen der Formel (II) und Formaldehyd geleitet werden. Für das erfindungsgemäße Verfahren wird Formaldehyd in wäßriger Lösung eingesetzt. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Säureadditions-Salze der Verbindungen der Formel (I) können in einfacher Weise nach üblichen Salzbildungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hoffmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.

Die erfindungsgemäßen Wirkstoffe der Formel (I) zeichnen sich durch eine hervorragende insektizide Wirksamkeit aus. Sie zeigen insbesondere beim Einsatz als Blattinsektizide und Bodeninsektizide eine hervorragende Wirkung gegen Maden wie z.B. Phorbia antiqua-Maden gegen Raupen, wie z. B. Plutella maculipennis, gegen Käferlarven, wie z.B. Phaedon cochleariae und Diabrotica balteata und Blattläuse, wie z.B. Myzus persicae und Aphis fabae.

Die neuen Verbindungen sind also besonders gut für einen Einsatz zur Bekämpfung von Blattinsekten und Bodeninsekten geeignet.

Weiterhin zeigen die neuen Verbindungen eine bakterizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden. Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikrooganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw, erreicht werden können. Sie zeigen insbesondere beim Einsatz als Ektoparasitizide eine ausgezeichnete Wirkung gegen Blowfly-larven wie z. B. Lucilia cuprina.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns.

Die biologische Wirksamkeit der erfindungsgemäßen Verbindungen soll anhand der folgenden Beispiele erläutert werden.

Herstellungsbeispiel

Beispiel 1

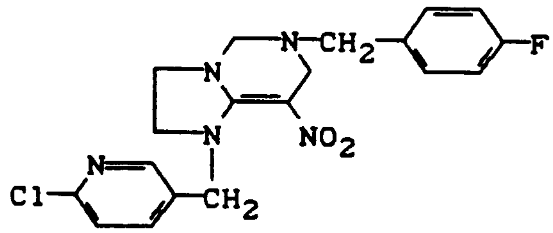

Zu einer Mischung aus 2,54 g (0,01 Mol) 3-(2-Chlor-pyridin-5-yl-methyl)-2-nitromethylen-imidazolidin und 1,25 g (0,01 Mol) 4-Fluorbenzylamin 90 ml Ethanol werden bei Raumtemperatur 1,5 ml (0,02 Mol) 37 % wäßrige Formaldehydlösung zugetropft und 3 Stunden unter Rückfluß erhitzt. Nach dem Abkühlen auf Raumtemperatur wird das Lösungsmittel im Vakuum entfernt, der Rückstand mit Ether versetzt und abgesaugt.

Man erhält 3,41 g (78 % der Theorie) 6,7-Dihydro-6-(4-fluorbenzyl)-8-nitro-(5H)-3-(2-chlorpyridin-5-yl-methyl)-imidazolidino-[2,3-f]-pyrimidin vom Schmelzpunkt 159 ° C.

Analog Beispiel 1 bzw. dem erfindungsgemäßen Verfahren können die in der nachfolgenden Tabelle 2 angegebenen Verbindungen der Formel (I) hergestellt werden:

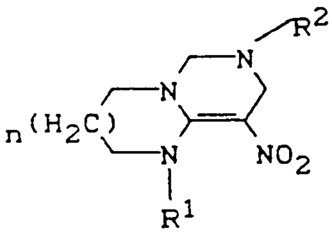

(I)

| Bsp. Nr. | n | $R^1$ | $R^2$ | physikal. Konstante |
|---|---|---|---|---|
| 2 | 1 | 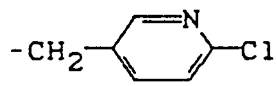 | 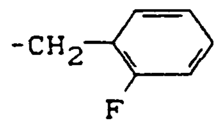 | Fp:156° C |
| 3 | 0 | 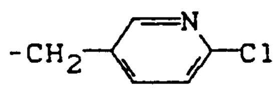 | 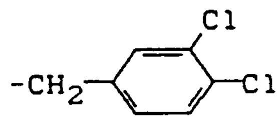 | Fp:168° C |
| 4 | 0 | 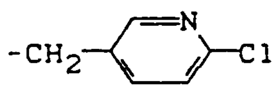 | 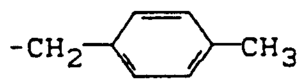 | Fp:146° C |
| 5 | 0 | 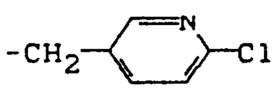 | 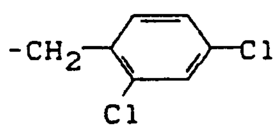 | Fp:139° C |
| 6 | 0 | 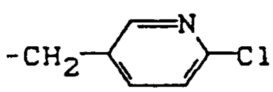 | 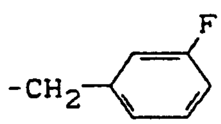 | Fp:124° C |
| 7 | 0 | 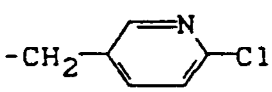 | 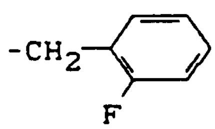 | |

| Bsp. Nr. | n | $R^1$ | $R^2$ | physikal. Konstante |
|---|---|---|---|---|
| 8 | 1 | 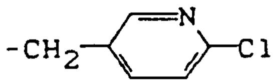 | 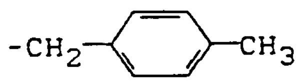 | Fp:132° C |
| 9 | 1 | 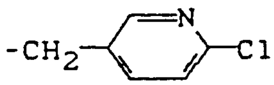 | 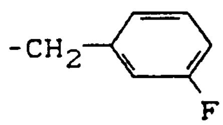 | Fp:146° C |
| 10 | 1 | 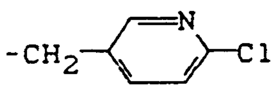 | 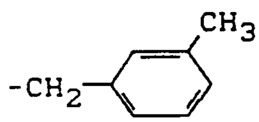 | Fp:135° C |
| 11 | 1 | 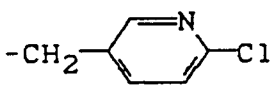 | 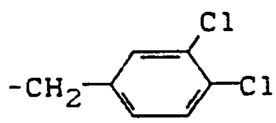 | Fp:149° C |
| 12 | 1 | 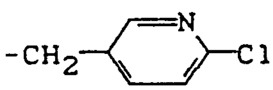 | 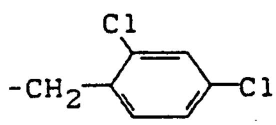 | Fp:91° C |
| 13 | 1 | 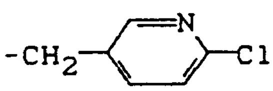 | 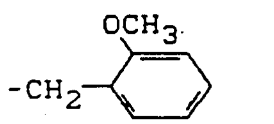 | Fp:154° C |
| 14 | 1 | 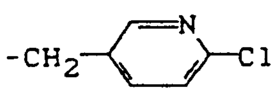 | 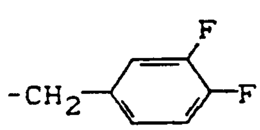 | Fp:142° C |

| Bsp. Nr. | n | $R^1$ | $R^2$ | physikal. Konstante |
|---|---|---|---|---|
| 15 | 0 | 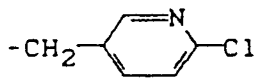 | 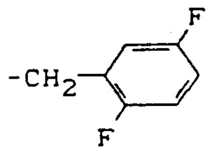 | Fp: 142° C |
| 16 | 1 | 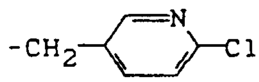 | 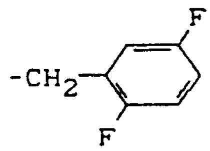 | Fp: 118° C |
| 17 | 1 | 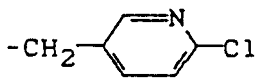 | 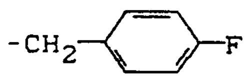 | Fp: 144° C |
| 18 | 1 | 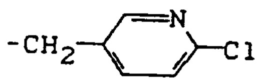 | 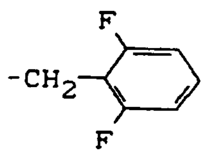 | Fp: 143° C |
| 19 | 0 | 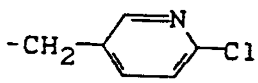 | 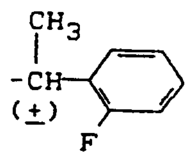 | Fp: 81° C |
| 20 | 1 | 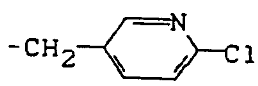 | 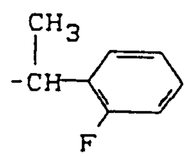 | Fp: 127° C |
| 21 | 1 | 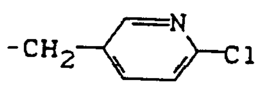 | 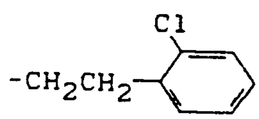 | Fp: 141° C |
| 22 | 0 | 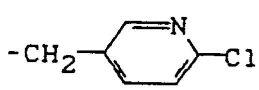 | 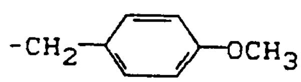 | Fp: 166° C |
| 23 | 1 | 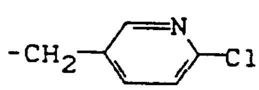 | 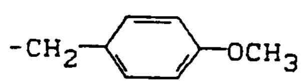 | Fp: 149° C |
| 24 | 0 | 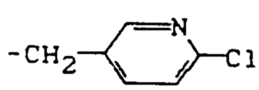 | 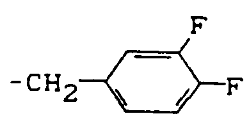 | Fp: 129° C |

| Bsp. Nr. | n | $R^1$ | $R^2$ | physikal. Konstante |
|---|---|---|---|---|
| 25 | 0 | 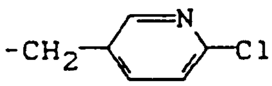 | 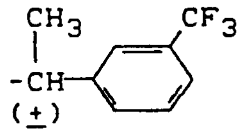 | Fp: 88° C |
| 26 | 1 | 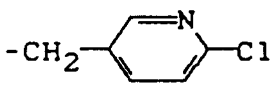 | 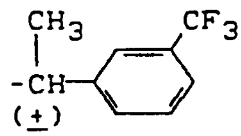 | Fp: 68° C |
| 27 | 0 | 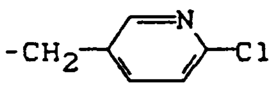 | 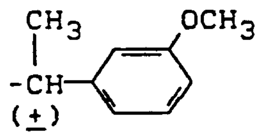 | Fp: 121° C |
| 28 | 1 | 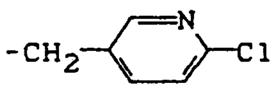 | 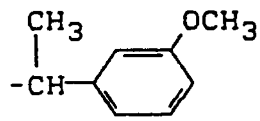 | Oel |
| 29 | 0 | 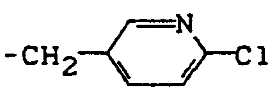 | 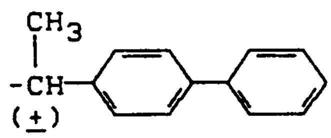 | Fp: 190° C |
| 30 | 1 | 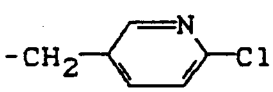 | 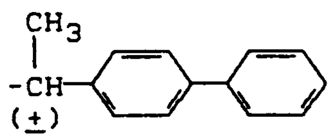 | Fp: 109° C |
| 31 | 0 | 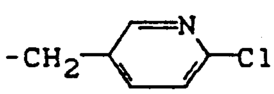 | 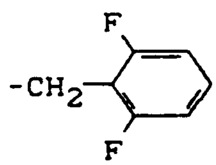 | Fp: 151° C |
| 32 | 1 | 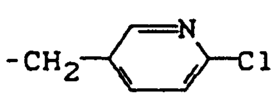 | 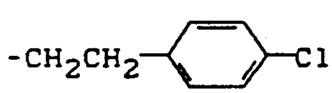 | Fp: 94° C |
| 33 | 1 | 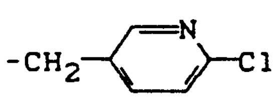 | 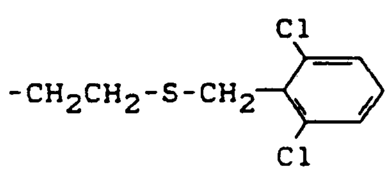 | Fp: 87° C |

| Bsp. Nr. | n | $R^1$ | $R^2$ | physikal. Konstante |
|---|---|---|---|---|
| 34 | 0 | 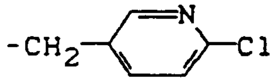 | 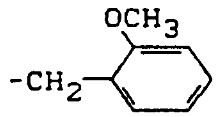 | Fp:122° C |
| 35 | 0 | 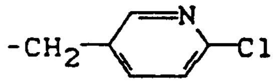 | 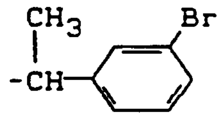 | Fp:116° C |
| 36 | 1 | 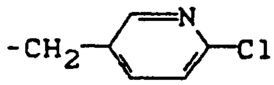 | 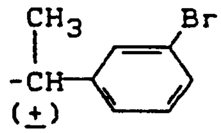 | Fp:127° C |
| 37 | 0 | 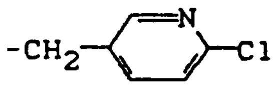 | 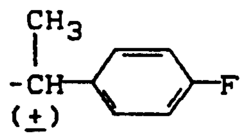 | Fp:132° C |
| 38 | 1 | 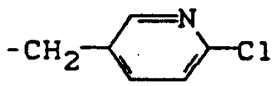 | 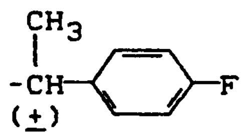 | Fp:170° C |
| 39 | 0 | 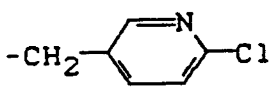 | 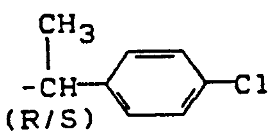 | Fp:123° C |
| 40 | 1 | 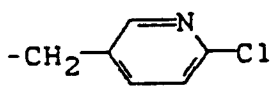 | 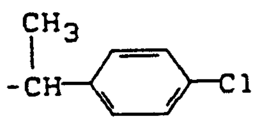 | Fp:129° C |
| 41 | 1 | 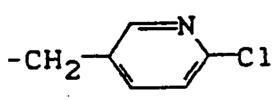 | 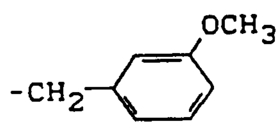 | Fp:146° C |
| 42 | 0 | 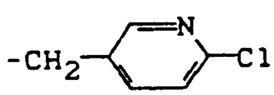 | 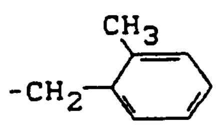 | Fp:129° C |

| Bsp. Nr. | n | $R^1$ | $R^2$ | physikal. Konstante |
|---|---|---|---|---|
| 43 | 0 | 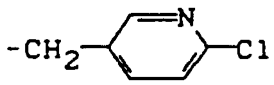 | 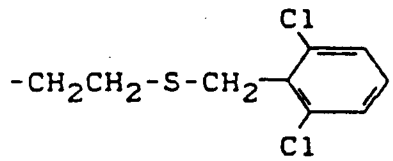 | Fp: 136° C |
| 44 | 1 | 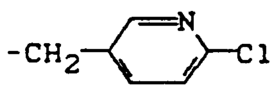 | 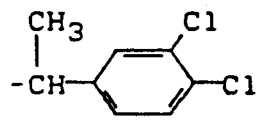 | Fp: 137° C |
| 45 | 1 | 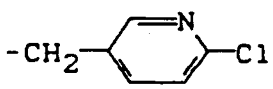 | 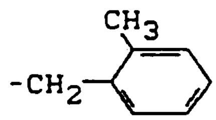 | Fp: 68° C |
| 46 | 0 | 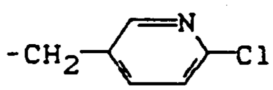 | 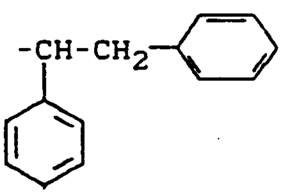 | Fp: 157° C |
| 47 | 1 | 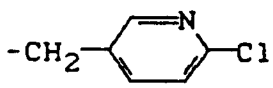 | 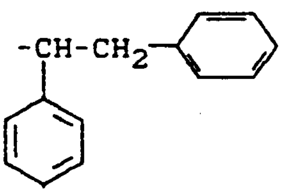 | Oel |
| 48 | 0 | 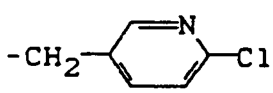 | 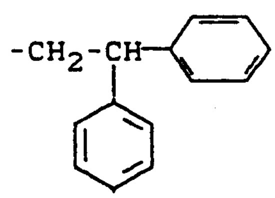 | Fp: 199° C |
| 49 | 1 | 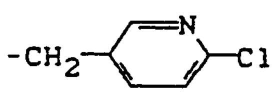 | 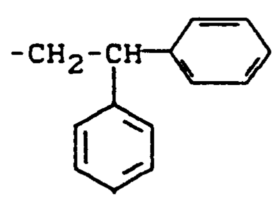 | Fp: 94° C |

| Bsp. Nr. | n | $R^1$ | $R^2$ | physikal. Konstante |
|---|---|---|---|---|
| 69 | 1 | 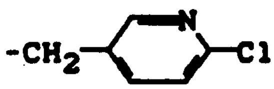 | 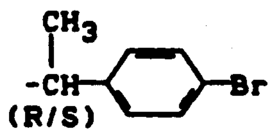 | Fp:157° C |
| 70 | 1 | 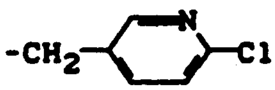 | 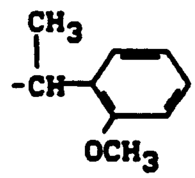 | Fp:132° C |
| 71 | 0 | 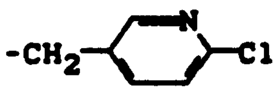 | 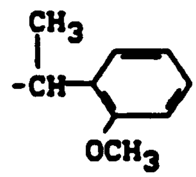 | Fp:111° C |
| 72 | 0 | 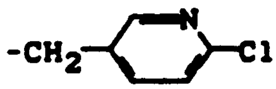 | 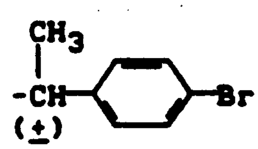 | Fp:118° C |
| 73 | 0 | 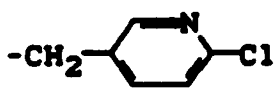 | 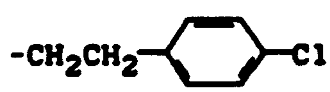 | Fp:146° C |
| 74 | 0 | 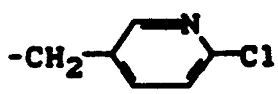 | 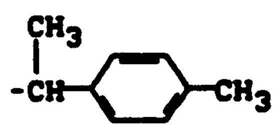 | Fp:139° C |

| Bsp. Nr. | n | $R^1$ | $R^2$ | physikal. Konstante |
|---|---|---|---|---|
| 56 | 0 | 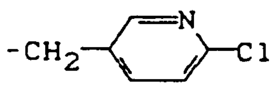 | 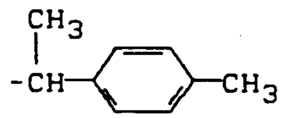 | Fp: 132° C |
| 57 | 0 | 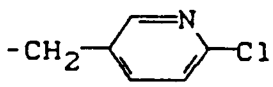 | 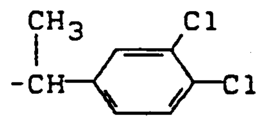 | Fp: 121° C |
| 58 | 0 | 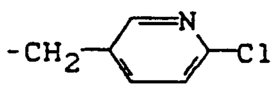 | 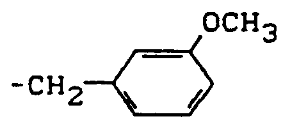 | Fp: 128° C |
| 59 | 0 | 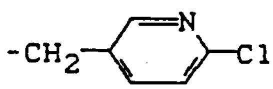 | 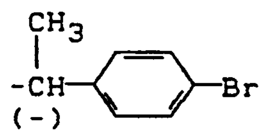 | Fp: 157° C |
| 60 | 0 | 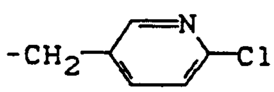 | 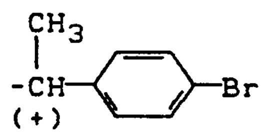 | Fp: 164° C |
| 61 | 0 | 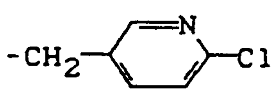 | 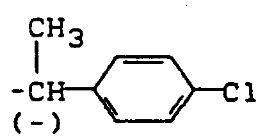 | Fp: 159° C |
| 62 | 0 | 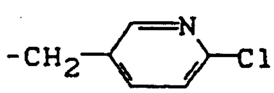 | 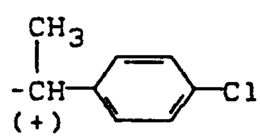 | Fp: 154° C |
| 63 | 1 | 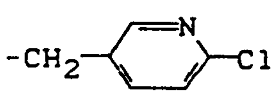 | 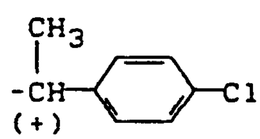 | Oel |

| Bsp. Nr. | n | $R^1$ | $R^2$ | physikal. Konstante |
|---|---|---|---|---|
| 64 | 0 | 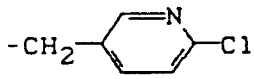 | 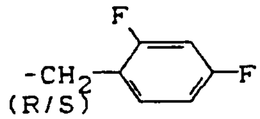 | Fp:138° C |
| 65 | 1 | 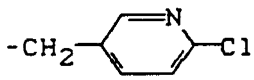 | 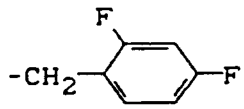 | Fp:166° C |
| 66 | 0 | 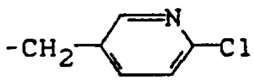 | 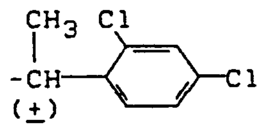 | Fp:174° C |
| 67 | 0 | 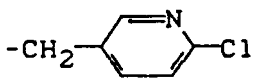 | 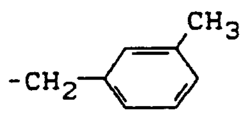 | Fp:124° C |
| 68 | 0 | 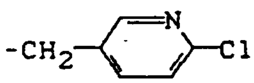 | 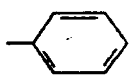 | Fp:189° C |
| 69 | 0 | 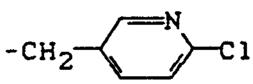 | 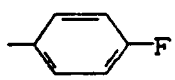 | Fp:121° C |
| 70 | 0 | 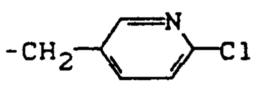 | 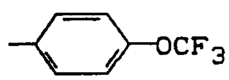 | Fp:182° C |
| 71 | 0 | 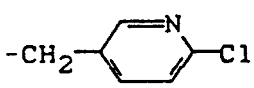 | 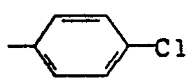 | Fp:193° C |
| 72 | 0 | H | 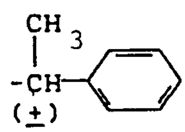 | Fp:155° C |
| 73 | 1 | H | 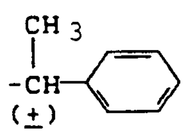 | Fp:149° C |
| 74 | 0 | 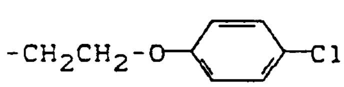 | 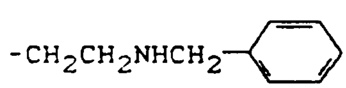 | Oel |

Beispiel C

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele 4, 7, 8 und 87 überlegene Wirkung gegenüber dem Stand der Technik:

Beispiel D

Grenzkonzentrations-Test / Bodeninsekten

Testinsekt: Phaedon cochleariae-Larven (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiel 4, 7, 8 und 87 überlegene Wirkung gegenüber dem Stand der Technik:

Beispiel E

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigt z.B. die folgende Verbindung der Herstellungsbeispiele 8 überlegene Wirksamkeit gegenüber dem Stand der Technik.

Beispiel F

Grenzkonzentrations-Test / Bodeninsekten

| | |
|---|---|
| Testinsekt: | Diabrotica balteata-Larven (im Boden) |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele 4, 7, 8 und 87 überlegene Wirksamkeit gegenüber dem Stand der Technik.

**Patentansprüche**

**1.** 1,2,3,4-Tetrahydro-5-nitropyrimidin-Derivate der Formel (I)

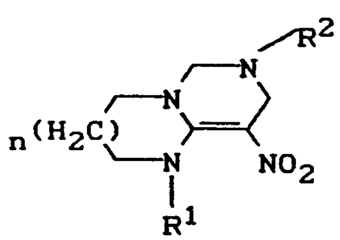

(I)

in welcher

| | |
|---|---|
| $R^1$ | für Wasserstoff oder für gegebenenfalls ein- bis dreifach gleich oder verschieden durch Halogen und/oder Cyano substituierte Reste aus der Reihe Benzyl, Picolyl (Pyridylmethyl) und Phenoxyalkyl steht, |
| n | für die Zahl 0 oder 1 steht und |
| $R^2$ | für die Gruppe $-A-R^3$ steht, in welcher |
| A | für eine direkte Bindung oder für die Gruppierungen $-(CH_2)_m-$ oder $-(CH_2)_x-Y-(CH_2)_z-$ steht, worin |
| m | für die Zahlen 1 bis 4 steht, |
| x und z | für die Zahlen 0, 1 oder 2 stehen, wobei |
| x und z | gleich oder verschieden sein können und |
| Y | für Sauerstoff, Schwefel oder für die Gruppierungen -NH- oder |

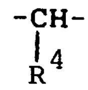

steht
worin

$R^4$ für gegebenenfalls durch Alkoxycarbonyl substituiertes $C_1$-$C_4$-Alkyl, Cyano, Hydroxy oder für Phenyl steht und

$R^3$ für gegebenenfalls einfach bis fünffach, gleich oder verschieden substituiertes Phenyl steht, wobei als Substituenten genannt seien Halogen, $C_{1-4}$-Alkyl, Halogen-$C_{1-2}$-alkyl, $C_{1-2}$-Alkoxy, Halogen-$C_{1-2}$-alkylthio, Hydroxy, Di-$C_{1-2}$-alkylamino, Carboxyl und Phenyl,

ausgenommen die Verbindungen, in denen

α)
$R^1$ für den Rest 2-Chlor-pyridin-5-yl-methyl und
$R^2$ gleichzeitig für die folgenden Reste steht: Benzyl, 4-Chlorbenzyl, 2-Chlorbenzyl, Phenethyl, 1-Phenylethyl und 3,4-Dimethoxy-benzyl, oder

β)
$R^1$ für Wasserstoff steht und gleichzeitig $R^2$ Benzyl und 4-Chlorbenzyl bedeutet,

und deren Säureadditionssalze.

**2.** 1,2,3,4-Tetrahydro-5-nitropyrimidin-Derivate der Formel (I)

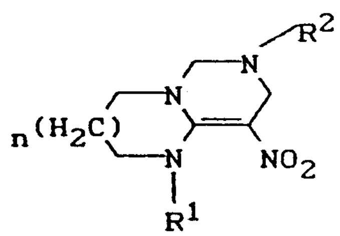

(I)

in welcher

$R^1$ für Wasserstoff oder für gegebenenfalls ein-bis dreifach gleich oder verschieden durch Halogen und/oder Cyano substituierte Reste aus der Reihe Benzyl, Picolyl (Pyridylmethyl) und Phenoxyalkyl steht,

n für die Zahl 0 oder 1 steht und

$R^2$ für die Gruppe -A-$R^3$ steht,
in welcher

A für eine direkte Bindung oder für die Gruppierung $-(CH_2)_x-Y-(CH_2)_z-$steht,
worin

x und z für die Zahlen 0, 1 oder 2 stehen, wobei
x und z gleich oder verschieden sein können und

Y für Sauerstoff, Schwefel oder für die Gruppierungen -NH- oder

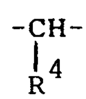

steht
worin

$R^4$ für $C_1$-$C_4$-Alkyl oder für Phenyl steht und

$R^3$ für gegebenenfalls durch Halogen oder Phenyl substituiertes Phenyl steht,
ausgenommen die Verbindungen, in denen

$R^1$ für den Rest 2-Chlor-pyridin-5-yl-methyl und

$R^2$ gleichzeitig für 1-Phenylethyl steht,
und deren Säureadditionssalze.

**3.** 1,2,3,4-Tetrahydro-5-nitropyrimidin-Derivate der Formel (I) gemäß Anspruch 1, in welcher

n fur die Zahl 0 steht,
$R^1$ für Wasserstoff oder für gegebenenfalls einfach bis dreifach, gleich oder verschieden durch Halogen und oder Cyano substituierte Reste aus der Reihe Benzyl, Picolyl und Phenoxy-$C_1$-$C_3$-alkyl steht,
$R^2$ für die Gruppe -A-$R^3$ steht, in welcher
A für die Gruppierung $-(CH_2)_x$-Y-$(CH_2)_z$- steht, worin
x und z für die Zahlen 0, 1 oder 2 stehen, wobei x und z gleich oder verschieden sein können und
Y für Sauerstoff, Schwefel oder für die Gruppierungen -NH- oder

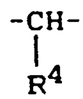

steht
worin
$R^4$ für $C_1$-$C_4$-Alkyl oder für Phenyl steht und
$R^3$ für gegebenefalls einfach bis fünffach durch Halogen oder Phenyl gleich oder verschieden substituiertes Phenyl steht,

ausgenommen Verbindungen, in denen $R^1$ für 2-Chlor-pyridin-5-yl-methyl und $R^2$ gleichzeitig für 1-Phenylethyl steht.

**4.** 1,2,3,4-Tetrahydro-5-nitro-pyrimidin-Derivate der Formel (I) gemäß Anspruch 1, in welcher

n für die Zahl 1 steht,
$R^1$ für Wasserstoff, gegebenenfalls einfach bis dreifach, gleich oder verschieden durch Halogen und/oder Cyano substituierte Reste aus der Reihe Benzyl, Picolyl und Phenoxy-$C_1$-$C_3$-alkyl steht,
$R^2$ für die Gruppe -A-$R^3$ steht, in welcher
A für die Gruppierung $-(CH_2)_x$-Y-$(CH_2)_z$- steht, worin
x und z für die Zahlen 0, 1 oder 2 stehen, wobeix und z gleich oder verschieden sein können und
Y für Sauerstoff, Schwefel oder für die Gruppierungen -NH- oder

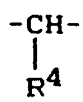

steht
worin
$R^4$ für $C_1$-$C_4$-Alkyl oder für Phenyl steht und
$R^3$ für Halogen-$C_1$-$C_3$-alkyl oder für gegebenenfalls einfach bis fünffach durch Halogen oder Phenyl gleich oder verschieden substituiertes Phenyl steht,

ausgenommen Verbindungen, in denen $R^1$ für 2-Chlor-pyridin-5-yl-methyl und $R^2$ gleichzeitig für 1-Phenylethyl steht.

**5.** 1,2,3,4-Tetrahydro-5-nitro-pyrimidin-Derivate der Formel (I) gemäß Anspruch 1, in welcher

n für die Zahl 0 oder 1 steht,

$R^1$ für Wasserstoff, gegebenenfalls einfach oder zweifach, gleich oder verschieden durch Fluor, Chlor, Brom und/oder Cyano substituierte Reste aus der Reihe Benzyl, Picolyl und Phenoxy-$C_1$-$C_2$-alkylsteht und

$R^2$ für gegebenenfalls einfach bis fünffach durch Halogen oder Phenyl gleich oder verschieden substituiertes Phenyl steht.

**6.** 1,2,3,4-Tetrahydro-5-nitro-pyrimidin-Derivate der Formel (I) gemäß Anspruch 1, in welcher

n für die Zahl 0 oder 1 steht,

$R^1$ für Wasserstoff, gegebenenfalls einfach oder zweifach, gleich oder verschieden durch Fluor, Chlor, Brom und/oder Cyano substituierte Reste aus der Reihe Benzyl, Picolyl und Phenoxy-$C_1$-$C_2$-alkylsteht und

$R^2$ für die Gruppe -A-$R^3$ steht, in welcher

A für die Gruppierung $-(CH_2)_x-Y-(CH_2)_z-$ steht, worin

x und z für die Zahlen 0, 1 oder 2 stehen, wobei x und z gleich oder verschieden sein können und

Y für Sauerstoff, Schwefel oder für die Gruppierungen -NH- oder

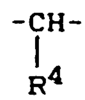

steht worin

$R^4$ für $C_1$-$C_2$-Alkyl oder für Phenyl steht und

$R^3$ für gegebenenfalls einfach bis fünffach durch Halogen oder Phenyl gleich oder verschieden substituiertes Phenyl steht.

**7.** Verfahren zur Herstellung von 1,2,3,4-Tetrahydro-5-nitro-pyrimidin-Derivaten der Formel (I)

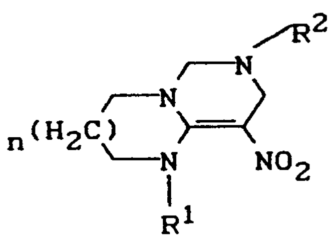

(I)

in welcher

$R^1$ für Wasserstoff oder für gegebenenfalls ein- bis dreifach gleich oder verschieden durch Halogen und/oder Cyano substituierte Reste aus der Reihe Benzyl, Picolyl (Pyridylmethyl) und Phenoxyalkyl steht,

n für die Zahl 0 oder 1 steht und

$R^2$ für die Gruppe -A-$R^3$ steht, in welcher

A für eine direkte Bindung oder für die Gruppierungen $-(CH_2)_m-$ oder $-(CH_2)_x-Y-(CH_2)_z-$ steht, worin

m für die Zahlen 1 bis 4 steht,

x und z für die Zahlen 0, 1 oder 2 stehen, wo

x und z gleich oder verschieden sein können und

Y für Sauerstoff, Schwefel oder für die Gruppierungen -NH- oder

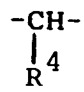

steht

worin

$R^4$ für gegebenenfalls durch Alkoxycarbonyl substituiertes $C_1$-$C_4$-Alkyl, Cyano, Hydroxy oder für Phenyl steht und

$R^3$ für gegebenenfalls einfach bis fünffach, gleich oder verschieden substituiertes Phenyl, steht, wobei als Substituenten genannt seien Halogen, $C_{1-4}$-Alkyl, Halogen-$C_{1-2}$-alkyl, $C_{1-2}$-alkoxy, Halogen-$C_{1-2}$-alkylthio, Hydroxy, Di-$C_{1-2}$-alkylamino, Carboxyl und Phenyl,

ausgenommen die Verbindungen, in denen

α)

$R^1$ für den Rest 2-Chlor-pyridin-5-yl-methyl und

$R^2$ gleichzeitig für die folgenden Reste steht: Benzyl, 4-Chlorbenzyl, 2-Chlorbenzyl, Phenethyl, 1-Phenylethyl und 3,4-Dimethoxybenzyl, oder

β)

$R^1$ für Wasserstoff steht und gleichzeitig Benzyl und 4-Chlorbenzyl bedeutet,

und von ihren Säureadditionssalzen,

dadurch gekennzeichnet, daß man Nitromethylen-Derivate der Formel (II)

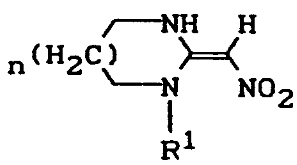 (II)

in welcher

$R^1$ und n die oben angegebenen Bedeutungen haben,

mit Aminen der Formel (III)

$R^3$-A-$NH_2$ (III)

in welcher

$R^3$ und A die oben angegebenen Bedeutungen haben,

in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt, und gegebenenfalls an die erhaltenen Verbindungen Säuren addiert.

**8.** Schädlingsbekämpfungsmittel, gekennzeichnet durch einen Gehalt an mindesens einem 1,2,3,4-Tetrahydro-5-nitro-pyrimidin-Derivat der Formel (I) gemäß Anspruch 1.

**9.** Insektizide Mittel, gekennzeichnet durch einen Gehalt an mindestens einem 1,2,3,4-Tetrahydro-5-nitro-pyrimidin-Derivat der Formel (I) gemäß Anspruch 1.

**10.** Verwendung von 1,2,3,4-Tetrahydro-5-nitro-pyrimidin-Derivaten der Formel (I) gemäß Anspruch 1 zur Bekämpfung von Insekten.

**11.** Verfahren zur Herstellung von Schädlingsbekämpfungsmitteln, dadurch gekennzeichnet, daß man 1,2,3,4-Tetrahydro-5-nitropyrimidin-Derivate der Formel (I) gemäß Anspruch 1 mit Streckmitteln und/oder oberflächenaktiven Mitteln vermischt.

## Claims

**1.** 1,2,3,4-Tetrahydro-5-nitropyrimidine derivatives of the formula (I)

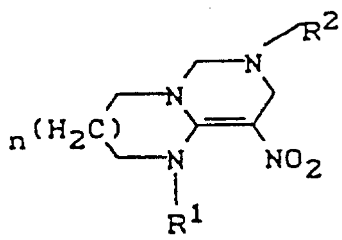

(I)

in which

$R^1$ represents hydrogen or radicals from the series comprising benzyl, picolyl (pyridylmethyl) and phenoxyalkyl, which are optionally monosubstituted, disubstituted or trisubstituted by identical or different halogen and/or cyano substituents,

n represents the number 0 or 1 and

$R^2$ represents the $-A-R^3$ group,
in which

A represents a direct bond or the $-(CH_2)_m-$ or $-(CH_2)_x-Y-(CH_2)_z-$ groupings,
where

m represents the numbers 1 to 4,

x and z represent the numbers 0, 1 or 2, it being possible for x and z to be identical or different, and

Y represents oxygen, sulphur or the -NH- or

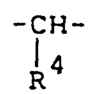

groupings,
where

$R^4$ represents optionally alkoxycarbonyl-substituted $C_1$-$C_4$-alkyl, cyano, hydroxyl or phenyl, and

$R^3$ represents phenyl which is optionally monosubstituted to pentasubstituted by identical or different substituents, substituents which may be mentioned being halogen, $C_{1-4}$-alkyl, halogeno-$C_{1-2}$-alkyl, $C_{1-2}$-alkoxy, halogeno-$C_{1-2}$-alkylthio, hydroxyl, di-$C_{1-2}$-alkylamino, carboxyl and phenyl,

with the exception of the compounds in which

α)
$R^1$ represents the 2-chloro-pyridin-5-yl-methyl radical and
$R^2$ simultaneously represents the following radicals: benzyl, 4-chlorobenzyl, 2-chlorobenzyl, phenethyl, 1-phenylethyl and 3,4-dimethoxybenzyl, or

β)
$R^1$ represents hydrogen and simultaneously $R^2$ denotes benzyl and 4-chlorobenzyl,

and acid addition salts thereof.

**2.** 1,2,3,4-Tetrahydro-5-nitropyrimidine derivatives of the formula (I)

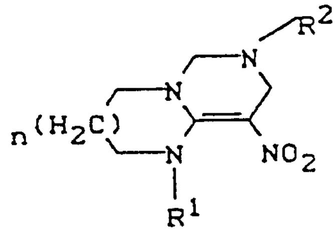

(I)

in which

| | |
|---|---|
| $R^1$ | represents hydrogen or radicals from the series comprising benzyl, picolyl (pyridylmethyl) and phenoxyalkyl, which are optionally monosubstituted, disubstituted or trisubstituted by identical or different halogen and/or cyano substituents, |
| n | represents the number 0 or 1 and |
| $R^2$ | represents the $-A-R^3$ group, in which |
| A | represents a direct bond or the $-(CH_2)_x-Y-(CH_2)_z$-grouping, where |
| x and z | represent the numbers 0, 1 or 2, it being possible for x and z to be identical or different, and |
| Y | represents oxygen, sulphur or the -NH- or 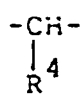 groupings, where |
| $R^4$ | represents $C_1$-$C_4$-alkyl or phenyl and |
| $R^3$ | represents phenyl which is optionally substituted by halogen or phenyl, with the exception of the compounds in which |
| $R^1$ | represents the 2-chloro-pyridin-5-yl-methyl radical and |
| $R^2$ | simultaneously represents 1-phenylethyl, |

and acid addition salts thereof.

**3.** 1,2,3,4-Tetrahydro-5-nitropyrimidine derivatives of the formula (I) according to Claim 1, in which

| | |
|---|---|
| n | represents the number 0, |
| $R^1$ | represents hydrogen or radicals from the series comprising benzyl, picolyl and phenoxy-$C_1$-$C_3$-alkyl, which are optionally monosubstituted, disubstituted or trisubstituted by identical or different halogen and/or cyano substituents, |
| $R^2$ | represents the $-A-R^3$ group, in which |
| A | represents the $-(CH_2)_x-Y-(CH_2)_z-$ grouping, where |
| x and z | represent the numbers 0, 1 or 2, it being possible for x and z to be identical or different, and |
| Y | represents oxygen, sulphur or the -NH- or 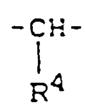 groupings, |

where
$R^4$ represents $C_1$-$C_4$-alkyl or phenyl and
$R^3$ represents phenyl which is optionally monosubstituted to pentasubstituted by identical or different substituents

with the exception of compounds in which $R^1$ represents 2-chloro-pyridin-5-yl-methyl and $R^2$ simultaneously represents 1-phenylethyl.

**4.** 1,2,3,4-Tetrahydro-5-nitropyrimidine derivatives of the formula (I) according to Claim 1, in which

n represents the number 1,
$R^1$ represents hydrogen or radicals from the series comprising benzyl, picolyl and phenoxy-$C_1$-$C_3$-alkyl, which are optionally monosubstituted, disubstituted or trisubstituted by identical or different halogen and/or cyano substituents,
$R^2$ represents the -A-$R^3$ group,
in which
A represents the $-(CH_2)_x$-Y-$(CH_2)_z$- grouping,
where
x and z represent the numbers 0, 1 or 2, it being possible for x and z to be identical or different, and
Y represents oxygen, sulphur or the -NH- or

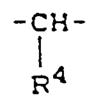

groupings,
where
$R^4$ represents $C_1$-$C_4$-alkyl or phenyl and
$R^3$ represents halogeno-$C_1$-$C_3$-alkyl or phenyl which is optionally monosubstituted to pentasubstituted by identical or different halogen or phenyl substituents

with the exception of compounds in which $R^1$ represents 2-chloro-pyridin-5-yl-methyl and $R^2$ simultaneously represents 1-phenylethyl.

**5.** 1,2,3,4-Tetrahydro-5-nitropyrimidine derivatives of the formula (I) according to Claim 1, in which

n represents the number 0 or 1,
$R^1$ represents hydrogen or radicals from the series comprising benzyl, picolyl and phenoxy-$C_1$-$C_2$-alkyl, which are optionally monosubstituted or disubstituted by identical or different fluorine, chlorine, bromine and/or cyano substituents, and
$R^2$ represents phenyl which is optionally monosubstituted to pentasubstituted by identical or different halogen or phenyl substituents.

**6.** 1,2,3,4-Tetrahydro-5-nitropyrimidine derivatives of the formula (I) according to Claim 1, in which

n represents the number 0 or 1,
$R^1$ represents hydrogen or radicals from the series comprising benzyl, picolyl and phenoxy-$C_1$-$C_2$-alkyl, which are optionally monosubstituted or disubstituted by identical or different fluorine, chlorine, bromine and/or cyano substituents, and
$R^2$ represents the -A-$R^3$ group,
in which
A represents the $-(CH_2)_x$-Y-$(CH_2)_z$- grouping,
in which
x and z represent the numbers 0, 1 or 2, it being possible for x and z to be identical or different, and
Y represents oxygen, sulphur or the -NH- or

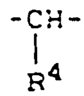

groupings,
in which

$R^4$ represents $C_1$-$C_2$-alkyl or phenyl, and

$R^3$ represents phenyl which is optionally monosubstituted to pentasubstituted by identical or different halogen or phenyl substituents.

**7.** Process for the preparation of 1,2,3,4-tetrahydro-5-nitro-pyrimidine derivatives of the formula (I)

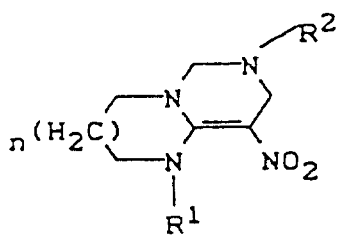

(I)

in which

$R^1$ represents hydrogen or radicals from the series comprising benzyl, picolyl (pyridylmethyl) and phenoxyalkyl, which are optionally monosubstituted, disubstituted or trisubstituted by identical or different halogen and/or cyano substituents,

n represents the number 0 or 1 and

$R^2$ represents the -A-$R^3$ group,
in which

A represents a direct bond or the $-(CH_2)_m-$ or $-(CH_2)_x-Y-(CH_2)_z-$ groupings,
where

m represents the numbers 1 to 4,

x and z represent the numbers 0, 1 or 2, it being possible for x and z to be identical or different, and

Y represents oxygen, sulphur or the -NH- or

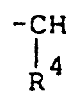

groupings,
where

$R^4$ represents optionally alkoxycarbonyl-substituted $C_1$-$C_4$-alkyl, cyano, hydroxyl or phenyl, and

$R^3$ represents phenyl which is optionally monosubstituted to pentasubstituted by identical or different substituents, substituents which may be mentioned being halogen, $C_{1-4}$-alkyl, halogeno-$C_{1-2}$-alkyl, $C_{1-2}$-alkoxy, halogeno-$C_{1-2}$-alkylthio, hydroxyl, di-$C_{1-2}$-alkylamino, carboxyl and phenyl,

with the exception of the compounds in which

α)

$R^1$ represents the 2-chloro-pyridin-5-yl-methyl radical and

$R^2$ simultaneously represents the following radicals: benzyl, 4-chlorobenzyl, 2-chlorobenzyl, phenethyl, 1-phenylethyl and 3,4-dimethoxybenzyl, or

β)

$R^1$ represents hydrogen and simultaneously denotes benzyl and 4-chlorobenzyl,

and acid addition salts thereof,
characterized in that nitromethylene derivatives of the formula (II)

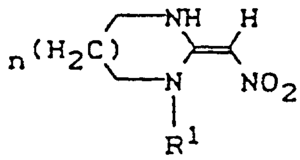

(II)

in which
$R^1$ and n have the abovementioned meanings,
are reacted with amines of the formula (III)

$R^3$-A-$NH_2$ (III)

in which
$R^3$ and A have the abovementioned meanings,
in the presence of at least twice the molar amount of formaldehyde, if appropriate in the presence of acid catalysts and if appropriate in the presence of diluents, and, if appropriate, the compounds obtained are subjected to an addition reaction.

**8.** Pesticides, characterized in that they contain at least one 1,2,3,4-tetrahydro-5-nitro-pyrimidine derivative of the formula (I) according to Claim 1.

**9.** Insecticides, characterized in that they contain at least one 1,2,3,4-tetrahydro-5-nitro-pyrimidine derivative of the formula (I) according to Claim 1.

**10.** Use of 1,2,3,4-tetrahydro-5-nitro-pyrimidine derivatives of the formula (I) according to Claim 1 for combating insects.

**11.** Process for the preparation of pesticides, characterized in that 1,2,3,4-tetrahydro-5-nitropyrimidine derivatives of the formula (I) according to Claim 1 are mixed with extenders and/or surface-active agents.

## Revendications

**1.** Dérivés de 1,2,3,4-tétrahydro-5-nitropyrimidine de formule (I)

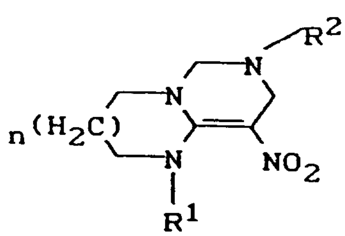

(I)

dans laquelle
- $R^1$ représente l'hydrogène ou des restes de la série benzyle, picolyle (pyridylméthyle) et phénoxyalkyle portant le cas échéant 1 à 3 substituants halogéno et/ou cyano identiques ou différentes,
- n représente le nombre 0 ou 1 et
- $R^2$ est le groupe -A-$R^3$,
  dans lequel
- A représente une liaison directe ou les groupements $-(CH_2)_m-$ ou $-(CH_2)_x-Y-(CH_2)_z-$, où
- m représente les nombres de 1 à 4,
- x et z représentent les nombres 0, 1 ou 2,

x et z pouvant être égaux ou différents

et

Y représente l'oxygène, le soufre ou les groupements -NH- ou

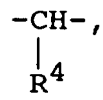

où

$R^4$ représente un groupe hydroxy, cyano, alkyle en $C_1$ à $C_4$ portant éventuellement un substituant alkoxycarbonyle, ou un groupe phényle, et

$R^3$ est un groupe phényle portant le cas échéant 1 à 5 substituants identiques ou différents, et on mentionne alors comme substituants des radicaux halogéno, alkyle en $C_1$ à $C_4$, halogénalkyle en $C_1$ ou $C_2$, alkoxy en $C_1$ ou $C_2$, halogénalkylthio en $C_1$ ou $C_2$, hydroxy, di-(alkyle en $C_1$ ou $C_2$)amino, carboxyle et phényle,

à l'exception des composés dans lesquels

α)

$R^1$ représente le reste 2-chloropyridine-5-yl-méthyle et

$R^2$ représente en même temps les restes suivants : benzyle, 4-chlorobenzyle, 2-chlorobenzyle, phénéthyle, 1-phényléthyle et 3,4-diméthoxybenzyle, ou bien

β)

$R^1$ représente l'hydrogène et $R^2$ désigne en même temps des groupes benzyle et 4-chlorobenzyle, et leurs sels d'addition d'acides.

**2.** Dérivés de 1,2,3,4-tétrahydro-5-nitropyrimidine de formule (I)

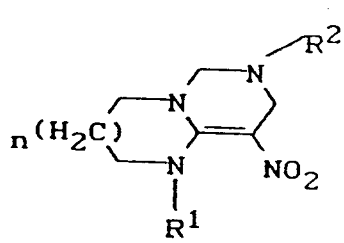

(I)

dans laquelle

$R^1$ représente l'hydrogène ou des restes de la série benzyle, picolyle (pyridylméthyle) et phénoxyalkyle portant le cas échéant 1 à 3 substituants halogéno et/ou cyano identiques ou différents,

n représente les nombres 0 ou 1 et

$R^2$ est le groupe $-A-R^3$,

dans lequel

A est une liaison directe ou représente le groupement $-(CH_2)_x-Y-(CH_2)_z-$,

dans lequel

x et z représentent les nombres 0, 1 ou 2,

x et z pouvant être égaux ou différents

et

Y représente l'oxygène, le soufre ou les groupements -NH- ou

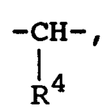

où

$R^4$ représente un groupe alkyle en $C_1$ à $C_4$ ou phényle et

$R^3$ est un groupe phényle portant éventuellement un substituant halogéno ou phényle,

à l'exception des composés dans lesquels :

$R^1$ représente le reste 2-chloropyridine-5-yl-méthyle et

$R^2$ est en même temps un groupe 1-phényléthyle,

et leurs sels d'addition d'acides.

**3.** Dérivés de 1,2,3,4-tétrahydro-5-nitropyrimidine de formule (I) suivant la revendication 1, dans laquelle

n représente le nombre 0,

$R^1$ représente l'hydrogène ou des restes de la série benzyle, picolyle et phénoxy-(alkyle en $C_1$ à $C_3$) portant éventuellement 1 à 3 substituants halogéno et/ou cyano identiques ou différents,

$R^2$ est le groupe -A-$R^3$,

dans lequel

A est le groupement $-(CH_2)_x-Y-(CH_2)_z-$,

où

x et z représentent les nombres 0, 1 ou 2,

x et z pouvant être égaux ou différents

et

Y représente l'oxygène, le soufre ou les groupements -NH- ou

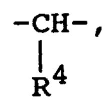

où

$R^4$ est un groupe alkyle en $C_1$ à $C_4$ ou phényle

et

$R^3$ est un groupe phényle portant le cas échéant 1 à 5 substituants halogéno ou phényle identiques ou différents,

à l'exception des composés dans lesquels :

$R^1$ est un groupe 2-chloropyridine-5-yl-méthyle et

$R^2$ est en même temps un groupe 1-phényléthyle.

**4.** Dérivés de 1,2,3,4-tétrahydro-5-nitropyrimidine de formule (I) suivant la revendication 1, dans laquelle

n représente le nombre 1,

$R^1$ représente l'hydrogène, des restes de la série benzyle, picolyle et phénoxy-(alkyle en $C_1$ à $C_3$) portant éventuellement 1 à 3 substituants halogéno et/ou cyano identiques ou différents,

$R^2$ est le groupe -A-$R^3$,

dans lequel

A représente le groupement $-(CH_2)_x-Y-(CH_2)_z-$,

dans lequel

x et z représentent les nombres 0, 1 ou 2,

x et z pouvant être égaux ou différents

et

Y représente l'oxygène, le soufre ou les groupements -NH- ou

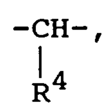

où

$R^4$ est un groupe alkyle en $C_1$ à $C_4$ ou phényle

et

$R^3$ est un groupe halogénalkyle en $C_1$ à $C_3$ ou un groupe phényle portant le cas échéant 1 à

5 substituants halogéno ou phényle identiques ou différents,
à l'exception de composés dans lesquels :
$R^1$ est un groupe 2-chloropyridine-5-yl-méthyle et
$R^2$ est en même temps un groupe 1-phényléthyle.

**5.** Dérivés de 1,2,3,4-tétrahydro-5-nitropyrimidine de formule (I) suivant la revendication 1, dans laquelle
n représente le nombre 0 ou 1,
$R^1$ représente l'hydrogène, des restes de la série benzyle, picolyle et phénoxy-(alkyle en $C_1$ ou $C_2$) portant le cas échéant 1 ou 2 substituants fluoro, chloro, bromo et/ou cyano identiques ou différents, et
$R^2$ est un groupe phényle portant le cas échéant 1 à 5 substituants halogéno ou phényle identiques ou différents.

**6.** Dérivés de 1,2,3,4-tétrahydro-5-nitropyrimidine de formule (I) suivant la revendication 1, dans laquelle
n représente le nombre 0 ou 1,
$R^1$ représente l'hydrogène, des restes de la série benzyle, picolyle et phénoxy-(alkyle en $C_1$ ou $C_2$) portant éventuellement 1 ou 2 substituants fluoro, chloro, bromo et/ou cyano identiques ou différents,
$R^2$ est le groupe $-A-R^3$,
dans lequel
A est le groupement $-(CH_2)_x-Y-(CH_2)_z-$,
dans lequel
x et z représentent les nombres 0, 1 ou 2,
x et z pouvant être égaux ou différents
et
Y représente l'oxygène, le soufre ou les groupements -NH- ou

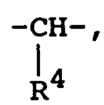

où
$R^4$ est un groupe alkyle en $C_1$ ou $C_2$ ou le groupe phényle et
$R^3$ est un groupe phényle portant le cas échéant 1 à 5 substituants halogéno ou phényle identiques ou différents.

**7.** Procédé de production de dérivés de 1,2,3,4-tétrahydro-5-nitropyrimidine de formule (I)

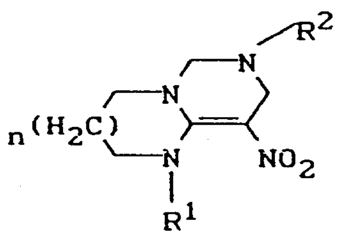

(I)

dans laquelle
$R^1$ représente l'hydrogène ou des restes de la série benzyle, picolyle (pyridylméthyle) et phénoxyalkyle portant le cas échéant 1 à 3 substituants halogéno et/ou cyano identiques ou différents,
n représente le nombre 0 ou 1 et
$R^2$ est le groupe $-A-R^3$,
dans lequel

A représente une liaison directe ou les groupements $-(CH_2)_m-$ ou $-(CH_2)_x-Y-(CH_2)_z-$, où
m représente les nombres de 1 à 4,
x et z représentent les nombres 0, 1 ou 2,
x et z pouvant être égaux ou différents
et
Y représente l'oxygène, le soufre ou les groupements -NH- ou

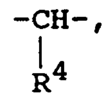

où
$R^4$ représente un groupe hydroxy, cyano, alkyle en $C_1$ à $C_4$ portant éventuellement un substituant alkoxycarbonyle, ou un groupe phényle, et
$R^3$ est un groupe phényle portant le cas échéant 1 à 5 substituants identiques ou différents, et on mentionne alors comme substituants des radicaux halogéno, alkyle en $C_1$ à $C_4$, halogénalkyle en $C_1$ ou $C_2$, alkoxy en $C_1$ ou $C_2$, halogénalkylthio en $C_1$ ou $C_2$, hydroxy, di-(alkyle en $C_1$ ou $C_2$)amino, carboxyle et phényle,

à l'exception des composés dans lesquels

α)
$R^1$ représente le reste 2-chloropyridine-5-yl-méthyle et
$R^2$ représente en même temps les restes suivants : benzyle, 4-chlorobenzyle, 2-chlorobenzyle, phénéthyle, 1-phényléthyle et 3,4-diméthoxybenzyle, ou bien

β)
$R^1$ représente l'hydrogène et $R^2$ désigne en même temps des groupes benzyle et 4-chlorobenzyle, et leurs sels d'addition d'acides,

et de leurs sels d'addition d'acides,

caractérisé en ce qu'on fait réagir des dérivés nitrométhyléniques de formule (II)

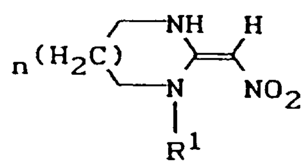

(II)

dans laquelle $R^1$ et n ont les définitions indiquées ci-dessus, avec des amines de formules (III)

$R^3-A-NH_2$ (III)

dans laquelle
$R^3$ et A ont les définitions indiquées ci-dessus,
en présence d'au moins le double de la quantité molaire de formaldéhyde, le cas échéant en présence de catalyseurs acides et en la présence éventuelle de diluants, et on additionne éventuellement des acides sur les composés obtenus.

**8.** Compositions pesticides, caractérisées par une teneur en au moins un dérivé de 1,2,3,4-tétrahydro-5-nitropyrimidine de formule (I) suivant la revendication 1.

**9.** Compositions insecticides caractérisées par une teneur en au moins un dérivé de 1,2,3,4-tétrahydro-5-nitropyrimidine de formule (I) suivant la revendication 1.

**10.** Utilisation de dérivés de 1,2,3,4-tétrahydro-5-nitropyrimidine de formule (I) suivant la revendication 1 pour combattre des insectes.

**11.** Procédé de préparation de compositions pesticides, caractérisé en ce qu'on mélange des dérivés de 1,2,3,4-tétrahydro-5-nitropyrimidine de formule (I) suivant la revendication 1 avec des diluants et/ou des

agents tensioactifs.